(19) Europäisches Patentamt
European Patent Office
Office européen des brevets

(11) Veröffentlichungsnummer: **0 180 835 B1**

# (12) EUROPÄISCHE PATENTSCHRIFT

(45) Veröffentlichungstag der Patentschrift: **06.09.89**

(51) Int. Cl.⁴: **C 07 D 249/08**, C 07 D 233/60, C 07 D 403/08, A 61 K 31/41, A 61 K 31/415, C 07 D 405/14, C 07 D 409/14

(21) Anmeldenummer: **85113279.5**

(22) Anmeldetag: **19.10.85**

(54) **Antimykotische Azolylcyclopropylcarbinolderivate.**

(30) Priorität: **02.11.84 DE 3440114**

(43) Veröffentlichungstag der Anmeldung: **14.05.86 Patentblatt 86/20**

(45) Bekanntmachung des Hinweises auf die Patenterteilung: **06.09.89 Patentblatt 89/36**

(84) Benannte Vertragsstaaten: **AT BE CH DE FR GB IT LI NL SE**

(56) Entgegenhaltungen:
**EP-A- 0 164 246**
**EP-A- 0 180 850**

(73) Patentinhaber: **BAYER AG, D-5090 Leverkusen 1 Bayerwerk (DE)**

(72) Erfinder: **Regel, Erik, Dipl.-Ing., Untere Bergerheide 26, D-5600 Wuppertal 1 (DE)**
Erfinder: **Böckmann, Klaus, Dr., Andreas-Gryphius-Strasse 7, D-5000 Köln 80 (DE)**
Erfinder: **Büchel, Karl Heinz, Prof. Dr., Dabringhausener Strasse 42, D-5093 Burscheid (DE)**
Erfinder: **Plempel, Manfred, Dr., Zwengenberger Strasse 3c, D-5657 Haan (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft die Verwendung von neuen substituierten Azolylcyclopropyl-azolylmethyl-carbinol-Derivaten als antimykotische Mittel, insbesondere als Antimykotika.

Es ist bereits allgemein bekannt geworden, daß bestimmte Diazolyl-Derivate, wie beispielsweise 1,3-Di-(1,2,4-triazol-1-yl)-2-(3-chlorphenyl)- bzw. –(4-chlorphenyl)- bzw. –phenyl-2-propanol, antimykotische Eigenschaften aufweisen (vergleiche EP-OS 0 044 605). Die Wirkung dieser Stoffe ist jedoch nicht immer in allen Indikationsbereichen voll zufriedenstellend.

Die am 11. 12. 1985 veröffentlichte Europäische Patentanmeldung EP-A 164 246 ist eine Publikation gemäß Art. 54 (3–4) EPÜ. Sie beschreibt Verbindungen der allgemeinen Formel

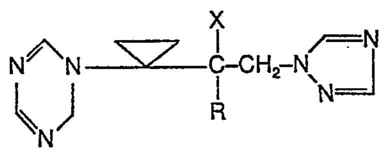

in der R für gegebenenfalls durch 1 bis 3 Substituenten aus der Gruppe F, Cl, Br, I, $CF_3$, $C_1$–$C_4$-Alkyl und $C_1$–$C_4$-alkoxy unabhängig voneinander substituiertes Phenyl oder 5-chloropyrid-2-yl steht und X für OH, F, Cl oder Br steht, sowie die O-Ester und O-Ether der Verbindungen, in denen X für OH steht.

Der Gegenstand dieser Anmeldung wird von den Ansprüchen der vorliegenden Erfindung nicht umfaßt.

Es wurde gefunden, daß die neuen substituierten Azolyl-cyclopropyl-azolylmethyl-carbinol-Derivate der Formel (I)

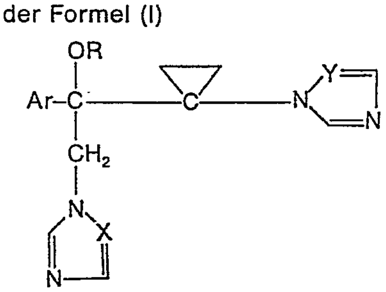

(I)

in welcher

a) X für CH oder N

Y für CH

Ar für gegebenenfalls einfach bis dreifach, insbesondere einfach oder zweifach, gleich oder verschieden substituiertes Phenyl steht, wobei als Substituenten genannt seine: Fluor, Chlor, Methyl, Isopropyl, tert.-Butyl, Methoxy, Methylthio, Trifluormethyl, Trifluormethoxy, Trifluormethylthio, sowie jeweils gegebenenfalls durch Fluor, Chlor und/oder Methyl substituiertes Phenyl oder Phenoxy, weiterhin für Naphthyl sowie für jeweils gegebenenfalls einfach oder zweifach, gleich oder verschieden substituiertes Furyl, Thienyl, Pyridinyl oder Pyrimidinyl steht, wobei als Substituenten die obengenannten Phenylsubstituenten in Frage kommen;

R[1] für Wasserstoff, Methyl, Ethyl, n-Propyl, Isopropyl, n-Butyl, Isobutyl, Allyl, Propargyl, Trimethylsilyl, Methylcarbonyl, Ethylcarbonyl, n-Propylcarbonyl, Isopropylcarbonyl, n-Butylcarbonyl, Isobutylcarbonyl, sowie für gegebenenfalls einfach bis dreifach, insbesondere einfach oder zweifach, gleich oder verschieden substituiertes Benzyl steht, wobei als Substituenten die bei Ar bereits genannten Phenylsubstituenten in Frage kommen;

oder

b) X für CH und

Y für N steht

und Ar, R[1] die unter a) genannte Bedeutung haben,

oder

c) X für N und

Y für N steht und

Ar für gegebenenfalls einfach bis dreifach, insbesondere einfach oder zweifach, gleich oder verschieden substituiertes Phenyl steht, wobei als Substituenten genannt seien: Fluor, Chlor, Methyl, Isopropyl, tert.-Butyl, Methoxy, Methylthio, Trifluormethyl, Trifluormethoxy, Trifluormethylthio, sowie jeweils gegebenenfalls durch Fluor, Chlor und/oder Methyl substituiertes Phenyl oder Phenoxy, weiterhin für Naphthyl sowie für jeweils gegebenenfalls einfach oder zweifach, gleich oder verschieden substituiertes Furyl, Thienyl, Pyridinyl oder Pyrimidinyl steht, wobei als Substituenten die obengenannten Phenylsubstituenten in Frage kommen;

mit Ausnahme von

4-Fluorphenyl, 4-Chlorphenyl, 4-Bromphenyl, 4-Iodphenyl, 4-Trifluormethylphenyl, 2-Chlorphenyl, 2,4-Dichlorphenyl, 2,4-Difluorphenyl, 2-Chlor-4-fluorphenyl, 2-Fluor-4-chlorphenyl, 2,5-Difluorphenyl, 4-Brom-2,5-difluorphenyl und 5-Chlorpyridin-2-yl,

R[1] für Wasserstoff, Methyl, Ethyl, n-Propyl, Isopropyl, n-Butyl, Isobutyl, Allyl, Propargyl, Trimethylsilyl, Methylcarbonyl, Ethylcarbonyl, n-Propylcarbonyl, Isopropylcarbonyl, n-Butylcarbonyl, Isobutylcarbonyl, sowie für gegebenenfalls einfach bis dreifach, insbesondere einfach oder zweifach, gleich oder verschieden substituiertes Benzyl steht, wobei als Substituenten die bei Ar bereits genannten Phenylsubstituenten in Frage kommen;

zur Anwendung bei der Behandlung von Krankheiten des menschlichen oder tierischen Körpers, sowie deren Säureadditions-Salze gute antimikrobielle, insbesondere antimykotische Eigenschaften, aufweisen.

Überraschenderweise zeigen die erfindungsgemäß zu verwendenden neuen substituierten Azolylcyclopropyl-azolylmethyl-carbinol-Derivate der Formel (I) in bestimmten Indikationsbereichen ein besseres Wirkungsspektrum als die aus dem Stand der Technik bekannten Diazolyl-Derivate 1,3-Di-(1,2,4-triazol-1-yl)-2-(3-chlorphenyl)- bzw. –(4-chlorphenyl)- bzw. –phenyl-2-propanol, welches konstitutionell und wirkungsmäßig naheliegende Verbindungen sind.

Bevorzugte erfindungsgemäße Verbindungen sind die Verbindungen, in denen X und Y für Stickstoff steht.

Zu den Säuren, die addiert werden können, gehören vorzugsweise Halogenwasserstoffsäuren,

wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z. B. p-Toluolsulfonsäure und 1,5-Naphthalindisulfonsäure.

Im einzelnen seien außer den bei der Herstellungsbeispielen genannten Verbindungen die folgenden Verbindungen der Formel (Ia) genannt:

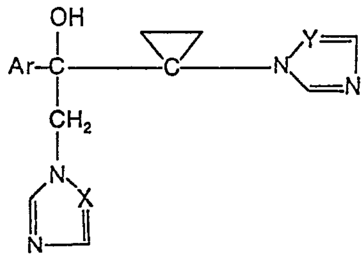

(Ia)

| Ar | X | Y |
|---|---|---|
| 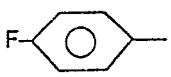 | N | CH |
| 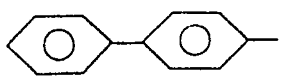 | N | CH |
| 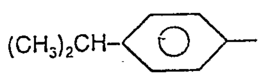 | N | CH |
| 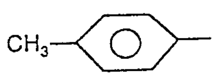 | N | CH |
| 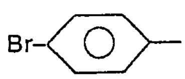 | N | CH |
| 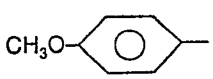 | N | CH |
| 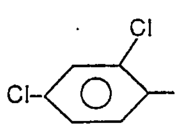 | N | CH |
| 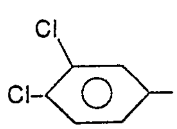 | N | CH |
| 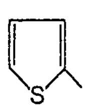 | N | CH |
| 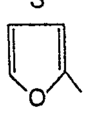 | N | CH |

| Ar | X | Y |
|---|---|---|
| 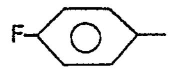 | CH | N |
| 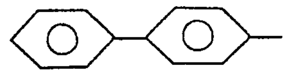 | CH | N |
| 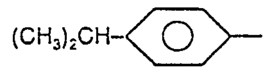 | CH | N |
| 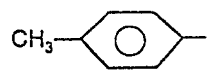 | CH | N |
| 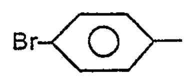 | CH | N |
| 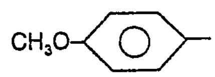 | CH | N |
| 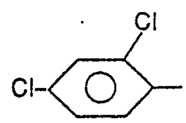 | CH | N |
| 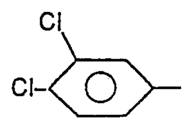 | CH | N |
| 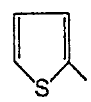 | CH | N |
| 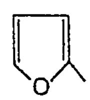 | CH | N |
| 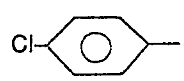 | CH | N |
| 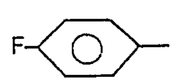 | CH | CH |
| 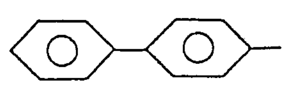 | CH | CH |
| 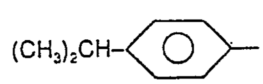 | CH | CH |
| 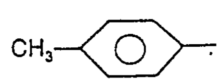 | CH | CH |
| 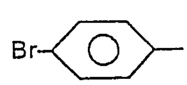 | CH | CH |

| Ar | X | Y |
|---|---|---|
| 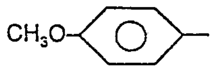 | CH | CH |
| 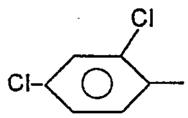 | CH | CH |
| 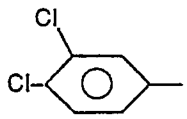 | CH | CH |
| 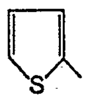 | CH | CH |
| 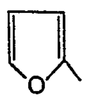 | CH | CH |
| 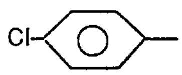 | CH | CH |
| 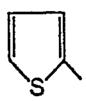 | N | N |
| 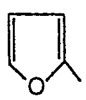 | N | N |

Die erfindungsgemäß zu verwendenden substituierten Azolylcyclopropyl-azolylmethyl-carbinol-Derivate sowie ihre Säureadditions-Salze sind noch nicht beschrieben. Sie können in allgemein bekannter Art und Weise erhalten werden, indem man

a) in einer ersten Stufe Aryl-azolylcyclopropyl-ketone der Formel (II)

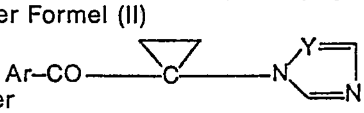 (II)

in welcher

Ar und Y die oben angegebene Bedeutung haben,

mit Dimethyloxosulfonium-methylid der Formel (III)

$$(CH_3)_2^{\delta+}SO^{\delta-}CH_2 \quad (III)$$

in Gegenwart eines Verdünnungsmittels, wie beispielsweise Dimethylsulfoxid, bei Temperaturen zwischen 20 °C und 80 °C umsetzt (vgl. hierzu J. Am. Chem. Soc. 87, 1363–1364 (1965)); und die dabei entstehenden Aryl-azolylcyclopropyl-oxirane der Formel (IV)

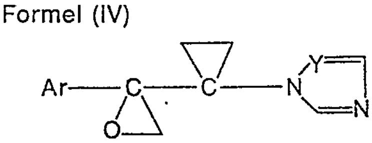 (IV)

in welcher

Ar und Y die oben angegebene Bedeutung haben,

in einer zweiten Stufe mit Azolen der Formel (V)

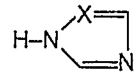 (V)

in welcher

X die oben agegebene Bedeutung hat,

in Gegenwart eines inerten organischen Lösungsmittels, wie beispielsweise Acetonitril oder Dimethylformamid, und in Gegenwart einer Base, wie beispielsweise Kaliumcarbonat oder Kaliumhydroxid, bei Temperaturen zwischen 60 und 150 °C umsetzt;

oder

b) Diazolyl-Keto-Derivate der Formel (VI)

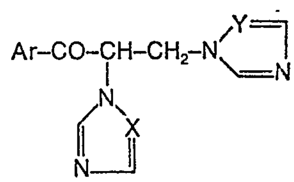 (VI)

in welcher

Ar, X und Y die oben angegebene Bedeutung haben,

mit Dimethyloxosulfonium-methylid der Formel (III)

$$(CH_3)_2^{\delta+}SO^{\delta-}CH_2 \quad : \quad (III)$$

entsprechend der ersten Stufe des Verfahrens (a) umsetzt;

und gegebenenfalls

c) die nach den Verfahren (a) oder (b) erhaltenen Hydroxy-Verbindungen der Formel (Ia)

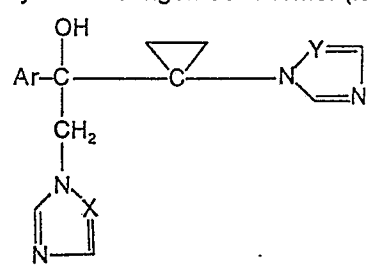 (Ia)

in welcher

Ar, X und Y die oben angegebene Bedeutung haben,

in Gegenwart eines Verdünnungsmittels in das Alkoholat überführt und dieses mit einem Halogenid der Formel (VII)

$$R^1\text{–Hal} \quad (VII)$$

in welcher

$R^1$ für Alkyl, Alkenyl, Alkinyl, Trialkylsilyl, gegebenenfalls substituiertes Phenylalkyl oder den Acyl-Rest steht und

Hal für Halogen steht,

in Gegenwart eines inerten organischen Lösungsmittels, wie beispielsweise Ether oder chlorierte Kohlenwasserstoffe, bei Temperaturen zwischen 20 und 100 °C umsetzt.

In einer bevorzugten Ausführungsform des Verfahrens (c) wird zweckmäßigerweise so verfahren, daß man von einer Hydroxy-Verbindung der Formel (Ia) ausgeht, letztere in einem geeigneten organischen Lösungsmittel mittels Alkalimetallhydrid oder Alkalimetall-amid in das Alkalimetallalkoholat überführt und letzteres ohne Isolierung sofort mit einem Halogenid der Formel (VII) umsetzt, wobei unter Austritt von Alkalihalogenid die erfindungsgemäßen Verbindungen der Formel (I) in einem Arbeitsgang erhalten werden.

Nach einer weiteren bevorzugten Ausführungsform des Verfahrens (c) werden zweckmäßigerweise die Herstellung der Alkoholate sowie die Alkylierung in einem Zweiphasensystem, wie beispielsweise wäßrige Natron- oder Kalilauge/Toluol oder Methylenchlorid, unter Zusatz von 0,01 bis 1 Mol eines Phasen-Transfer-Katalysators, wie beispielsweise Ammonium- oder Phosphoniumverbindungen, durchgeführt, wobei in der organischen Phase oder an der Grenzfläche die Alkoholate und mit den in der organischen Phase befindlichen Halogeniden umgesetzt werden.

Die Säureadditionssalze der Verbindungen der Formel (I) können in enfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Aryl-azolylcyclopropyl-ketone der Formel (II) sind neu. Sie werden erhalten, indem man Aryl-halogenpropyl-ketone der Formel (VIII)

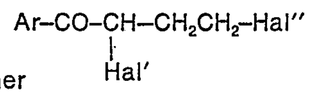
(VIII)

in welcher

Ar die oben angegebene Bedeutung hat und

Hal' und Hal'' für Halogen, vorzugsweise Brom oder Chlor stehen,

mit Azolen der Formel (V)

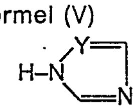
(V)

in welcher

Y die oben angegebene Bedeutung hat,

entsprechend der zweiten Stufe des Verfahrens (a) umsetzt.

Die Aryl-halogenpropyl-ketone der Formle (VIII) sind bekannt (vergleiche z.B. DE-OS 2 521 104, DE-OS 2 320 355 und DE-OS 2 351 948); bzw. können sie in allgemein üblicher Weise erhalten werden.

Das außerdem für die erste Stufe des Verfahrens (a) und für das Verfahren (b) als Ausgangsstoff zu verwendende Dimethyloxosulfoniummethylid der Formel (III) wird bei den obigen Umsetzungen in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, in Gegenwart eines Verdünnungsmittels erzeugt.

Die weiterhin für die zweite Stufe des Verfahrens (a) als Ausgangsstoffe zu verwendenden Azole der Formel (V) sind allgemein bekannte Verbindungen der organischen Chemie.

Die Diazolyl-Keto-Derivate der Formel (VI) sind neu. Sie werden erhalten, indem man Aryl-azolylmethyl-ketone der Formel (IX)

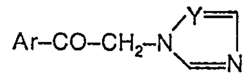
(IX)

in welcher

Ar und Y die oben angegebene Bedeutung haben,

mit Hydroxymethylazolen der Formel (X)

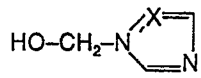
(X)

in welcher

X die oben angegebene Bedeutung hat,

in Gegenwart eines inerten organischen Verdünnungsmittels, wie beispielsweise Toluol, und in Gegenwart eines Katalysators, wie beispielsweise Piperidinacetat, vorzugsweise bei der Siedetemperatur des verwendeten Lösungsmittels umsetzt.

Die Aryl-azolylmethyl-ketone der Formel (IX) sind bekannt (vergleiche z.B. DE-OS 2 431 407, 2 610 022 und 2 638 470). Ebenso bekannt sind die Hydroxymethylazole der Formel (X) (vergleiche EP 0 006 102 sowie Chem. Heterocycl. Comp. 1980, 189).

Die außerdem für das Verfahren (b) als Ausgangsstoffe zu verwendenden Halogenide der Formel (VII) sind allgemein bekannte Verbindungen der organischen Chemie.

Die erfindungsgemäß verwendbaren Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bi-phasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trychophyton mentagrophytes, Microsporon-Arten, wie Microsporon felineum sowie Torulopsis-Arten, wie Torulopsis glabrate. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:

Dermatomykosen und Systemmykosen durch Trichophyton mentagrophytes und andere Trichophytonarten, Microsporonarten, Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder $^1/_2$, $^1/_3$ oder $^1/_4$ einer Einzeldosis enthalten. Eine Einzeldosis enthält vorzugsweise die Mengen Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzukker, Glucose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenen Überzügen und Hüllen versehen sein und so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentione, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsverzögerer und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Olivenöl, Ricinusöl und Sesamöl, Glycerin, Glycerinformal, Tetrahydrofurfurylalkohol, Polyethylenglykole und Fettsäureester des Sorbitan oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylalkohol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe, sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 2,5 bis etwa 200, vorzugsweise 5 bis 150 mg/kg Körpergewicht je 24 Stunden, gegebenenfalls in Form mehrerer Einzelgaben zur Erzielung der gewünschten Ergebnisse zu verabreichen.

Bei oraler Applikation werden die erfindungsgemäßen Wirkstoffe in Gesamtmengen von etwa 2,5 bis etwa 200, vorzugsweise 5 bis 150 mg/kg Körpergewicht je 24 Stunden und bei parenteraler Applikation in Gesamtmengen von etwa 2,5 bis etwa 50, vorzugsweise 1 bis 25 mg/kg Körpergewicht je 24 Stunden verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben aufgeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele:
Beispiel 1

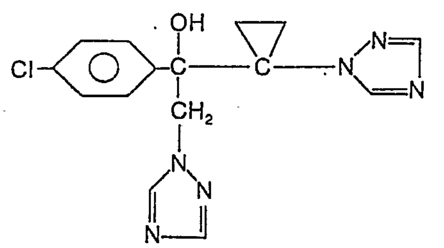

(I-1)

(Verfahren a)

1. Stufe

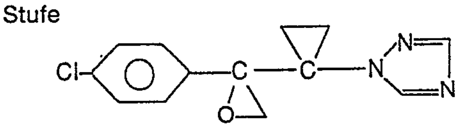

(IV-1)

Zu einer Mischung aus 3,7 g Natriumhydrid (80%ig) und 26,1 g Trimethylsulfoxoniumiodid tropft man bei 10 °C 100 ml trockenes Dimethylsulfoxid zu und läßt 1 Stunde bei Raumtemperatur nachrühren. Anschließend versetzt man tropfenweise mit 24 g 1-(4-Chlorbenzoyl)-1-(1,2,4-triazol-1-yl)-cyclopropan in 50 ml Dimethylsulfoxid. Das Reaktionsgemisch wird zwei Tage bei Raumtemperatur gerührt. Danach gießt man auf 600 ml Eiswasser, extrahiert mehrmals mit Essigester, wäscht die vereinigten organischen Phasen mit Wasser, trocknet über Natriumsulfat und engt im Vakuum ein. Man erhält 27,2 g rohes 1-[1-(4-Chlorphenyl)-oxiranyl]-1-(1,2,4-triazol-1-yl)-cyclopropan als gelbliches Öl, das direkt weiter umgesetzt wird. Brechungsindex $n_D^{20}$: 1.5635.

2. Stufe

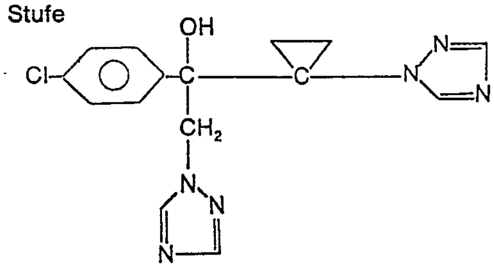

(I-1)

18 g rohes 1-[1-(4-Chlorphenyl)-oxiranyl]-1-(1,2,4-triazol-1-yl)-cyclopropan (Bsp. IV-1), 9,5 g Kaliumcarbonat und 15 g 1,2,4-Triazol werden in 100 ml Acetonitril acht Stunden unter Rückfluß erhitzt. Das Reaktionsgemisch wird anschließend im Vakuum eingeengt und in einem Gemisch aus Wasser und Methylenchlorid aufgenommen. Die organische Phase wird abgetrennt, mit Wasser gewaschen, über Natriumsulfat getrocknet und im Vakuum eingeengt. Der Rückstand wird mit Toluol verrührt, abgesaugt und aus 300 ml Ethanol umkristallisiert. Man erhält 10 g (44% der Theorie) 1-(4-Chlorphenyl)-1-[1-(1,2,4-triazol-1-yl)-cyclopropyl]-2-(1,2,4-triazol-1-yl)-1-ethanol vom Schmelzpunkt 211 °C.

Herstellung des Ausgangsproduktes

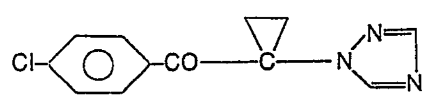

(II-1)

Zu einer siedenden Lösung von 100 g Kaliumcarbonat und 110 g 1,2,4-Triazol in 400 ml Aceton tropft man eine Lösung von 150 g 4-Chlorphenyl-(1-brom-3-chlorpropyl)-keton in 200 ml Aceton und rührt anschließend 8 Stunden unter Rückfluß. Danach wird im Vakuum eingeengt und der Rückstand in 500 ml Wasser verrührt. Der ausfallende Niederschlag wird abgesaugt, mehrmals mit Wasser gewaschen und getrocknet. Man erhält 104 g (82% der Theorie) 1-(4-Chlorbenzoyl)-1-(1,2,4-triazol-1-yl)-cyclopropan vom Schmelzpunkt 78 °C.

Verfahren b)

Zu einem Gemisch von 24,2 g Trimethylsulfoxoniumiodid und 12,3 g Kalium-tert.-butylat in 60 ml Dimethylsulfoxid wird eine Lösung von 15,1 g 1-(4-Chlorphenyl)-2,3-di-(1,2,4-triazol-1-yl)-propanon in 75 ml Dimethylsulfoxid zugetropft. Man läßt das Reaktionsgemisch 18 Stunden bei Raumtemperatur rühren. Anschließend wird im Vakuum eingeengt; der Rückstand wird in Methylenchlorid gelöst, mit Wasser gewaschen, über Natriumsulfat getrocknet und eingeengt. Der Rückstand wird chromatographisch (Kieselgel/Methylenchlorid) gereinigt. Das verbleibende Öl wird durch Verrühren mit Ether zur Kristallisation gebracht. Man erhält 6,3 g (38,2% der Theorie) 1-(4-Chlorphenyl)-1-[1-(1,2,4-triazol-1-yl)-cyclopropyl]-2-(1,2,4-triazol-1-yl)-1-ethanol vom Schmelzpunkt 208 °C.

Herstellung des Ausgangsproduktes

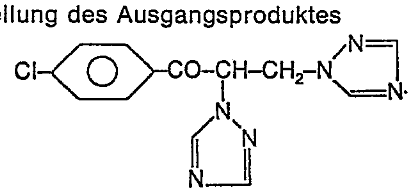 (VI-1)

Ein Gemisch aus 44,3 g 4-Chlorphenyl-(1,2,4-triazol-1-yl-methyl)-keton, 19,6 g 1-Hydroxymethyl-1,2,4-triazol, 300 ml Toluol, 6 g Essigsäure und 2 ml Piperidin wird am Wasserabscheider bis zur beendeten Wasserabscheidung erhitzt. Man läßt erkalten, saugt den entstandenen kristallinen Niederschlag ab und wäscht ihn mit Diisopropylether nach. Man erhält 47 g (76% der Theorie) 1-(4-Chlorphenyl)-2,3-di-(1,2,4-triazol-1-yl)-1-propanon vom Schmelzpunkt 204 °C.

In analoger Weise und entsprechend diesen Verfahren können die in der nachfolgenden Tabelle 1 aufgeführten erfindungsgemäßen Verbindungen der Formel (I)

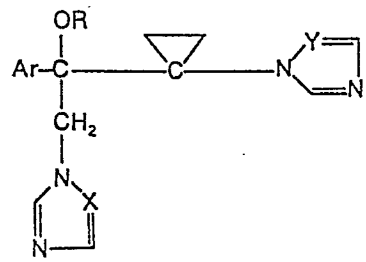 (I)

erhalten werden:

| Bsp. Nr. | Ar | R | X | Y | Schmelzpunkt (°C) |
|---|---|---|---|---|---|
| I-3 | 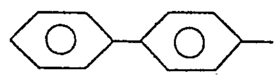 | H | N | N | 184 |
| I-4 | 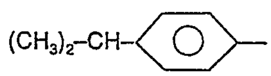 | H | N | N | 167 |
| I-5 | 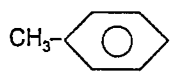 | H | N | N | 148 |
| I-6 | 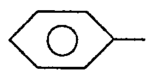 | H | N | N | 146 |
| I-9 | 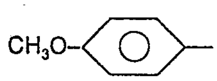 | H | N | N | 178 |
| I-10 | 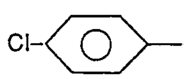 | H | N | CH | 196 |
| I-11 | 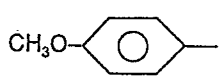 | H | H | N | 213 |

Entsprechend Beispiel 1 und gemäß den angegebenen Verfahrensbedingungen können die in der nachfolgenden Tabelle 2 aufgeführten Vorprodukte der Formel (II)

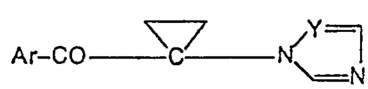 (II)

erhalten werden:

| Bsp.-Nr. | Ar | Y | Schmelzpunkt (°C) |
|---|---|---|---|
| II-3 | 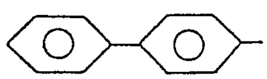 | N | 169 |
| II-4 | 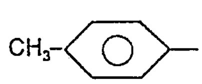 | N | 107 |

| Bsp.-Nr. | Ar | Y | Schmelzpunkt (°C) |
|---|---|---|---|
| II-5 | 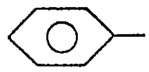 | N | 78–81 |
| II-8 | 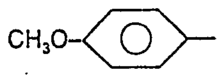 | N | 90 |
| II-9 | 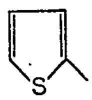 | N | 91 |

Entsprechend Beispiel 1 und gemäß den angegebenen Verfahrensbedingungen können die in der nachfolgenden Tabelle 3 aufgeführten Zwischenprodukte der Formel (IV)

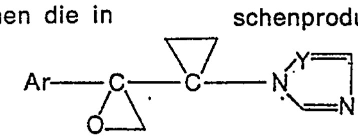 (IV)

erhalten werden:

| Bsp.-Nr. | Ar | Y | Physikalische Konstante |
|---|---|---|---|
| IV-3 | 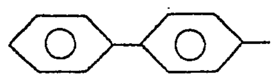 | N | Öl |
| IV-4 | 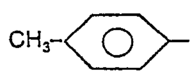 | N | Öl |
| IV-5 | 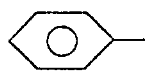 | N | Öl |
| IV-8 | 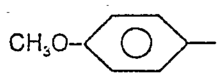 | N | Öl |

Entsprechend Beispiel 1 und gemäß den angegebenen Verfahrensbedingungen können die in der nachfolgenden Tabelle 4 aufgeführten Vorprodukte der Formel (VI)

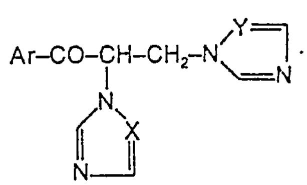 (VI)

erhalten werden:

| Bsp.-Nr. | Ar | X | Y | Schmelzpunkt (°C) |
|---|---|---|---|---|
| VI-4 | 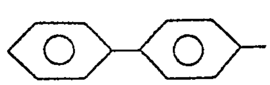 | N | N | 195 |
| VI-5 | 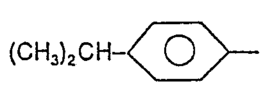 | N | N | 170 |

| Bsp.-Nr. | Ar | X | Y | Schmelzpunkt (°C) |
|---|---|---|---|---|
| VI-6 | 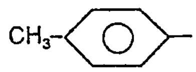 | N | N | 212 |
| VI-7 | 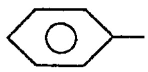 | N | N | 170 |

Verwendungsbeispiele

In dem in-vitro-Test werden die nachstehend angegebenen, aus der EP-OS 0 044 605 bekannten Verbindungen als Vergleichssubstanzen eingesetzt:

(A) 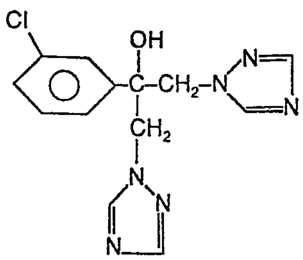

(B) 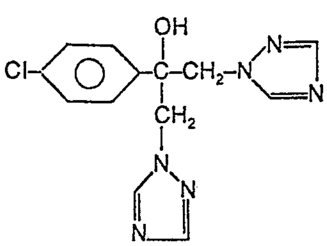

(C) 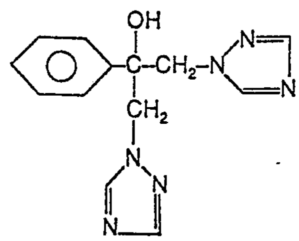

Beispiel A

Antimykotische in-vitro-Wirksamkeit

Versuchsbeschreibung

Die in-vitro-Prüfungen wurden im Reihenverdünnungstest mit Keiminokula von durchschnittlich $5 \times 10^3$ bis $10^4$ Keimen/ml Substrat durchgeführt. Als Nährmedium dienten

a) für Dermatophyten und Schimmelpilze: Sabourand's milieu d'épreuve

b) für Hefen: Fleischextrakt-Traubenzucker-Bouillon.

Die Bebrütungstemperatur betrug 28 bis 37 °C, die Bebrütungsdauer lag bei 24 bis 96 Stunden bei Hefen und 96 Stunden bei Dermatophyten und Schimmelpilzen.

In diesem Test zeigten z.B. die erfindungsgemäßen Verbindungen 1, 3, 6 und 7 eine bessere antimykotische Wirkung als die aus dem Stand der Technik bekannten Verbindungen (A), (B) und (C).

Tabelle A

Antimykotische in-vitro-Wirksamkeit

| Wirkstoff | MHK-Werte in mcg/ml Nährmedium | | | | |
|---|---|---|---|---|---|
| | Trichophyton mentagr. | Microsporum canis | Candida albicans | Torulopsis glabrata | Aspergillus fumigatus |
| (A) (bekannt) | 32 | – | >64 | >64 | >64 |
| (B) (bekannt) | 16 | – | 2 | 64 | >64 |
| (C) (bekannt) | 64 | – | 64 | >64 | >64 |
| Verbindungen gemäss Herstellungs-Bsp.: | | | | | |
| I-3 | <1 | 4 | 2 | 8 | 8 |
| I-6 | 4 | 32 | 8 | >64 | 32 |

Beispiel B

Antimykotische in-vitro-Wirksamkeit (oral) bei Mäusecandidose

Versuchsbeschreibung:

Mäuse vom Typ SPF-$CF_1$ wurden intravenös mit $1–2 \times 10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 10–100 mg/kg Körpergewicht der Präparate oral behandelt.

Ergebnis:

Unbehandelte Tiere starben am 3. bis 6. Tag post infektionem. Die Überlebensrate am 6. Tag post infektionem betrug bei unbehandelten Kontrolltieren etwa 5%.

In diesem Test zeigen z. B. die erfindungsgemäßen Verbindungen 2, 3, 5 und 6 gute bis sehr gute Wirkung, d.h. >60% Überlebende am 6. Tag p.i.

Tabelle B
Antimykotische in-vivo-Wirkung (oral) bei Mäusecandidose

| Wirkstoff | Wirkung |
|---|---|
| I-2 | ***** |
| I-3 | *** |
| I-5 | **** |
| I-6 | ***** |

Zeichenerklärung:

| | | |
|---|---|---|
| ***** | = sehr gute Wirkung | = 90% Überlebende am 6. Tag p.i. |
| **** | = gute Wirkung | = 80% Überlebende am 6. Tag p.i. |
| *** | = Wirkung | = 60% Überlebende am 6. Tag p.i. |
| ** | = schwache Wirkung | = 40% Überlebende am 6. Tag p.i. |
| * | = Spur Wirkung | = unter 40% Überlebende am 6. Tag p.i. |
| k.W. | | = kein Unterschied zur unbehandelten Infektionskontrolle. |

**Patentansprüche**

1. Azolylcyclopropyl-azolylmethyl-carbinol-Derivate der allgemeinen Formel

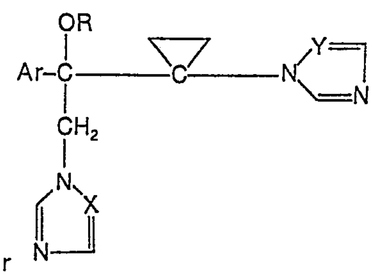

(I)

in welcher

a) X für CH oder N
Y für CH
Ar für gegebenenfalls einfach bis dreifach, insbesondere einfach oder zweifach, gleich oder verschieden substituiertes Phenyl steht, wobei als Substituenten genannt seien: Fluor, Chlor, Methyl, Isopropyl, tert.-Butyl, Methoxy, Methylthio, Trifluormethyl, Trifluormethoxy, Trifluormethylthio, sowie jeweils gegebenenfalls durch Fluor, Chlor und/oder Methyl substituiertes Phenyl oder Phenoxy, weiterhin für Naphthyl sowie für jeweils gegebenenfalls einfach oder zweifach, gleich oder verschieden substituiertes Furyl, Thienyl, Pyridinyl oder Pyrimidinyl steht, wobei als Substituenten die obengenannten Phenylsubstituenten in Frage kommen;

$R^1$ für Wasserstoff, Methyl, Ethyl, n-Propyl, Isopropyl, n-Butyl, Isobutyl Allyl, Propargyl, Trimethylsilyl, Methylcarbonyl, Ethylcarbonyl, n-Propylcarbonyl, Isopropylcarbonyl, n-Butylcarbonyl, Isobutylcarbonyl, sowie für gegebenenfalls einfach bis dreifach, insbesondere einfach oder zweifach, gleich oder verschieden substituiertes Benzyl steht, wobei als Substituenten die bei Ar bereits genannten Phenylsubstituenten in Frage kommen;
oder

b) X für CH und
Y für N steht
und Ar, $R^1$ die unter a) genannte Bedeutung haben
oder

c) X für N und
Y für N steht und
Ar für gegebenenfalls einfach bis dreifach, insbesondere einfach oder zweifach, gleich oder verschieden substituiertes Phenyl steht, wobei als Substituenten genannt seien: Fluor, Chlor, Methyl, Isopropyl, tert.-Butyl, Methoxy, Methylthio, Trifluormethyl, Trifluormethoxy, Trifluormethylthio, sowie jeweils gegebenenfalls durch Fluor, Chlor und/oder Methyl substituiertes Phenyl oder Phenoxy, weiterhin für Naphthyl sowie für jeweils gegebenenfalls einfach oder zweifach, gleich oder verschieden substituiertes Furyl, Thienyl, Pyridinyl oder Pyrimidinyl steht, wobei als Substituenten die obengenannten Phenylsubstituenten in Frage kommen;
mit Ausnahme von

4-Fluorphenyl, 4-Chlorphenyl, 4-Bromphenyl, 4-Iodphenyl, 4-Trifluormethylphenyl, 2-Chlorphenyl, 2,4-Dichlorphenyl, 2,4-Difluorphenyl, 2-Chlor-4-fluorphenyl, 2-Fluor-4-chlorphenyl, 2,5-Difluorphenyl, 4-Brom-2,5-difluorphenyl und 5-Chlorpyridin-2-yl,

$R^1$ für Wasserstoff, Methyl, Ethyl, n-Propyl, Isopropyl, n-Butyl, Isobutyl, Allyl, Propargyl, Trimethylsilyl, Methylcarbonyl, Ethylcarbonyl, n-Propylcarbonyl, Isopropylcarbonyl, n-Butylcarbonyl, Isobutylcarbonyl, sowie für gegebenenfalls einfach bis dreifach, insbesondere einfach oder zweifach, gleich oder verschieden substituiertes Benzyl steht, wobei als Substituenten die bei Ar bereits genannten Phenylsubstituenten in Frage kommen;
zur Anwendung bei der Behandlung von Krankheiten des menschlichen oder tierischen Körpers.

2. 1-(4-Phenylphenyl)-1-[1-(1,2,4-triazol-1-yl)-cyclopropyl]-2-[1,2,4-triazol-1-yl)-1-ethanol zur Anwendung bei der Bekämpfung von Krankheiten des menschlichen oder tierischen Körpers.

3. Arzneimittel enthaltend mindestens eine Verbindung der allgemeinen Formel (I) gemäß Anspruch 1 oder die Verbindung gemäß Anspruch 2.

4. Verfahren zur Herstellung von Arzneimitteln gemäß Anspruch 3, dadurch gekennzeichnet, daß man den oder die Wirkstoffe gegebenenfalls unter Verwendung von üblichen Hilfs- und/oder Trägerstoffen in eine geeignete Applikationsform überführt.

**Claims**

1. Azolylcyclopropyl-azolylmethyl-carbinol derivatives of the general formula (I)

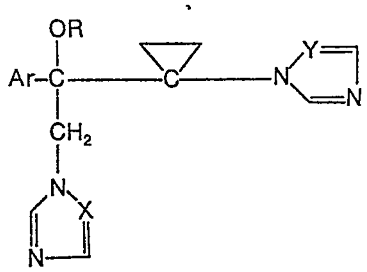

(I)

in which

a) X represents CH or N
Y represents CH
Ar represents phenyl which optionally has one to three, in particular one or two, identical or different substituents, the substituents which may be mentioned being: fluorine, chlorine, methyl, isopropyl, tert.-butyl, methoxy, methylthio, trifluoromethyl, trifluoromethoxy, trifluoromethylthio, and phenyl or phenoxy each of which is optionally substituted by fluorine, chlorine and/or methyl, furthermore represents naphthyl and represents furyl, thienyl, pyridinyl or pyrimidinyl, each of which optionally has one or two, identical or different, substituents, suitable substituents being the abovementioned phenyl substituents;
$R^1$ represents hydrogen, methyl, ethyl, n-propyl, isopropyl, n-butyl, isobutyl, allyl, propargyl, trimethylsilyl, methylcarbonyl, ethylcarbonyl, n-propylcarbonyl, isopropylcarbonyl, n-butylcarbonyl, isobutylcarbonyl, and represents benzyl which optionally has one to three, in particular one or two, identical or different substituents, suitable substituents being the phenyl substituents already mentioned for Ar;

or

b) X represents CH and
Y represents N
and Ar and $R^1$ have the meaning mentioned under a)

or

c) X represents N and
Y represents N and
Ar represents phenyl which optionally has one to three, in particular one or two, identical or different substituents, the substituents which may be mentioned being: fluorine, chlorine, methyl, isopropyl, tert.-butyl, methoxy, methylthio, trifluoromethyl, trifluoromethoxy, trifluoromethylthio, and phenyl or phenoxy each of which is optionally substituted by fluorine, chlorine and/or methyl, furthermore represents naphthyl and represents furyl, thienyl, pyridinyl or pyrimidinyl, each of which optionally has one or two, identical or different, substituents, suitable substituents being the abovementioned phenyl substituents;
with the exception of
4-fluorophenyl, 4-chlorophenyl, 4-bromophenyl, 4-iodophenyl, 4-trifluoromethylphenyl, 2-chlorophenyl, 2,4-dichlorophenyl, 2,4-difluorophenyl, 2-chloro-4-fluorophenyl, 2-fluoro-4-chlorophenyl, 2,5-difluorophenyl, 4-bromo-2,5-difluorophenyl and 5-chloropyridin-2-yl,
$R^1$ represents hydrogen, methyl, ethyl, n-propyl, isopropyl, n-butyl, isobutyl, allyl, propargyl, trimethylsilyl, methylcarbonyl, ethylcarbonyl, n-propylcarbonyl, isopropylcarbonyl, n-butylcarbonyl, isobutylcarbonyl, and represents benzyl which optionally has one to three, in particular one or two, identical or different substituents, suitable substituents being the phenyl substituents already mentioned for Ar;

for use in the treatment of diseases of the human and animal body.

2. 1-(4-Phenylphenyl)-1-[1-(1,2,4-triazol-1-yl)-cyclopropyl]-2-(1,2,4-triazol-1-yl)-2-ethanol for use in combating diseases of the human and animal body.

3. Medicaments containing at least one compound of the general formula (I) according to Claim 1 or the compound according to claim 2.

4. Process for the preparation of medicaments according to Claim 3, characterised in that the active compound or compounds are converted into a suitable form for administration, where appropriate with the use of customary auxiliaries and/or excipients.

**Revendications**

1. Dérivés d'azolylcyclopropyl-azolylméthyl-carbinols de formule générale:

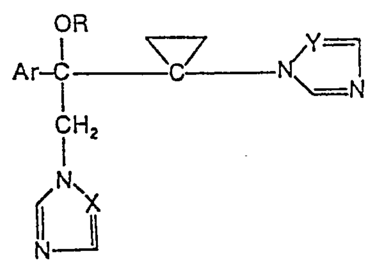

(I)

dans laquelle

a) X représente CH ou N
Y représente CH,
Ar représente un groupe phényle éventuellement substitué une à trois fois, en particulier, une ou deux fois, de manière identique ou différente; comme substituants, on mentionnera: l'atome de fluor, l'atome de chlore, le groupe méthyle, le groupe isopropyle, le groupe tert.-butyle, le groupe méthoxy, le groupe méthylthio, le groupe trifluorométhyle, le groupe trifluorométhoxy, le

groupe trifluorométhylthio; ainsi qu'un groupe phénoxy ou un groupe phényle éventuellement substitué par l'atome de fluor, l'atome de chlore et/ou le groupe méthyle; de même qu'un groupe naphthyle, ainsi qu'un groupe pyrimidinyle, le groupe pyridinyle, le groupe thiényle ou le groupe furyle éventuellement substitué chacun une ou deux fois de manière identique ou différente, en envisageant, comme substituants, les substituants du groupe phényle précités:

$R^1$ représente l'atome d'hydrogène, le groupe méthyle, le groupe éthyle, le groupe n-propyle, le groupe isopropyle, le groupe n-butyle, le groupe isobutyle, le groupe allyle, le groupe propargyle, le goupe triméthylsilyle, le groupe méthylcarbonyle, le groupe éthylcarbonyle, le groupe n-propyl-carbonyle, le groupe isopropyl-carbonyle, le groupe n-butyl-carbonyle, le groupe isobutyl-carbonyle, ainsi que le groupe benzyle éventuellement substitué une à trois fois, en particulier, une ou deux fois, de manière identique ou différente, en envisageant, comme substituants, les substituants du groupe phényle déjà mentionnés pour Ar;

ou

b) X représente CH et

Y représente N,

tandis que Ar, $R^1$ ont les significations mentionnées sub a)

ou

c) X représente N, et

Y représente N, et

Ar représente un groupe phényle éventuellement substitué une à trois fois, en particulier, une ou deux fois, de manière identique ou différente; comme substituants, on mentionnera: l'atome de fluor, l'atome de chlore, le groupe méthyle, le groupe isopropyle, le groupe tert.-butyle, le groupe méthoxy, le groupe méthylthio, le groupe trifluorométhyle, le groupe trifluorométhoxy, le groupe trifluorométhylthio; ainsi qu'un groupe phénoxy ou un groupe phényle éventuellement substitué chacun par l'atome de fluor, l'atome de chlore et/ou le groupe méthyle; de même qu'un groupe naphthyle, ainsi qu'un groupe pyrimidinyle, le groupe pyridinyle, le groupe thiényle ou le groupe furyle éventuellement substitué chacun une ou deux fois, de manière identique ou différente, en envisageant, comme substituants, les substituants précités du groupe phényle;

à l'exception

du groupe 4-fluorophényle, du groupe 4-chlorophényle, du groupe 4-bromophényle, du groupe 4-iodophényle, du groupe 4-trifluorométhylphényle, du groupe 2-chlorophényle, du groupe 2,4-dichlorophényle, du groupe 2,4-difluorophényle, du groupe 2-chloro-4-fluorophényle, du groupe 2-fluoro-4-chlorophényle, du groupe 2,5-difluorophényle, du groupe 4-bromo-2,5-difluorophényle et du groupe 5-chloropyridin-2-yle,

$R^1$ représente l'atome d'hydrogène, le groupe méthyle, le groupe éthyle, le groupe n-propyle, le groupe isopropyle, le groupe n-butyle, le groupe isobutyle, le groupe allyle, le groupe propargyle, le groupe triméthylsilyle, le groupe méthylcarbonyle, le groupe éthyl-carbonyle, le groupe n-propyl-carbonyle, le groupe isopropyl-carbonyle, le groupe n-butyl-cabonyle, le groupe isobutyl-carbonyle, de même le groupe benzyle éventuellement substitué une à trois, en particulier, une ou deux fois, de manière identique ou différente, en envisageant, comme substituants, les substituants du groupe phényle déjà mentionnés pour Ar;

en vue de les utiliser lors du traitement de maladies du corps humain ou animal.

2. 1-(4-phénylphenyl)-1-[2-(1,2,4-triazol-1-yl)-cyclopropyl]-2-(1,2,4-triazol-1-yl)-1-éthanol en vue de l'appliquer pour combattre des maladies du corps humain ou animal.

3. Médicament contenant au moins un composé de formule générale (I) selon la revendication 1 ou le composé selon la revendication 2.

4. Procédé de préparation de médicaments selon la revendication 3, caractérisé en ce qu'on transforme la ou les substances actives sous une forme d'application appropriée en utilisant éventuellement des substances auxiliaires et/ou des substances supports habituelles.